Europäisches Patentamt

European Patent Office

Office européen des brevets

(11) Veröffentlichungsnummer: **0 269 092 B1**

# EUROPÄISCHE PATENTSCHRIFT

(45) Veröffentlichungstag der Patentschrift: **10.03.93**

(21) Anmeldenummer: **87117411.6**

(22) Anmeldetag: **25.11.87**

Die Akte enthält technische Angaben, die nach dem Eingang der Anmeldung eingereicht wurden und die nicht in dieser Patentschrift enthalten sind.

(51) Int. Cl.5: **G01N 33/531**, G01N 33/543, G01N 33/552, //G01N33/78, G01N33/576, G01N33/68

(54) **Verfahren zur Bestimmung einer spezifisch bindefähigen Substanz.**

(30) Priorität: **26.11.86 DE 3640412**

(43) Veröffentlichungstag der Anmeldung:
**01.06.88 Patentblatt 88/22**

(45) Bekanntmachung des Hinweises auf die Patenterteilung:
**10.03.93 Patentblatt 93/10**

(84) Benannte Vertragsstaaten:
**AT BE CH DE ES FR GB GR IT LI LU NL SE**

(56) Entgegenhaltungen:
**EP-A- 0 101 912
EP-A- 0 142 193
DE-A- 2 738 183
US-A- 4 572 901**

(73) Patentinhaber: **BOEHRINGER MANNHEIM GMBH
Patentabteilung, Abt. E Sandhofer Strasse 112-132 Postfach 31 01 20
W-6800 Mannheim 31 Waldhof(DE)**

(72) Erfinder: **Tischer, Wilhelm, Dr.
Finkenweg 5
W-8123 Peissenberg(DE)**
Erfinder: **Maier, Josef
Schmädlstrasse 15
W-8120 Weilheim(DE)**
Erfinder: **Deeg, Rolf, Dr.
Hirtenstrasse 7
W-8139 Bernried(DE)**

(74) Vertreter: **Huber, Bernhard, Dipl.-Chem. et al
Patentanwälte H. Weickmann, Dr. K. Fincke F.A. Weickmann, B. Huber Dr. H. Liska, Dr. J. Prechtel Kopernikusstrasse 9 Postfach 86 08 20
W-8000 München 86 (DE)**

**Beschreibung**

Die Erfindung betrifft ein Verfahren zur Bestimmung einer spezifisch bindefähigen Proteinsubstanz nach dem Prinzip des Immunoassay, wobei einer der Partner des miteinander spezifisch bindefähigen Substanzpaares festphasengebunden vorliegt, sowie ein dazu geeignetes Trägermaterial.

Zur Bestimmung einer spezifisch bindefähigen Substanz bedient man sich häufig Verfahren nach dem Prinzip des Immunoassay. Dabei wird einer der Partner eines spezifisch miteinander bindefähigen Substanzpaares mit dem für ihn spezifischen Rezeptor, der in an sich bekannter Weise markiert ist, umgesetzt. Das Konjugat aus diesen beiden Substanzen kann dann noch mit einem Rezeptor, der für das Konjugat oder eines der beiden Teile des Konjugats spezifisch ist, umgesetzt werden. Für diese immunologischen Verfahren gibt es viele Variationen. Vorteilhaft ist es dabei, wenn einer der Rezeptoren an eine Festphase gebunden vorliegt. Dies erleichtert die Trennung von gebunden und nicht gebunden vorliegenden Reaktionspartnern. Zur Bestimmung der spezifisch bindefähigen Substanz wird dann die Menge an an der Festphase gebundenem markiertem Reaktionspartner oder an in der Lösung vorliegendem markiertem Reaktionspartner gemessen und zu der Menge an zu bestimmendem Reaktionspartner in an sich bekannter Weise in Beziehung gesetzt.

Als Festphase werden bei den immunologischen Verfahren üblicherweise Kunststoffröhrchen oder Mikrotiterplatten, an deren Innenoberfläche der Reaktionspartner fixiert ist, oder Kugeln, an deren Außenoberfläche der Reaktionspartner fixiert ist, verwendet. Diese Kunststoffröhrchen, Mikrotiterplatten oder Kugeln bestehen üblicherweise aus relativ inertem Kunststoffmaterial, so daß die Bindung des Reaktionspartners Schwierigkeiten bereitet. Darüberhinaus muß die Bindung des spezifischen Reaktionspartners an die jeweilige Oberfläche so erfolgen, daß er nicht die Fähigkeit zur spezifischen Bindung der für ihn spezifisch bindefähigen Substanz verliert. Aus diesem Grunde erfolgt die Bindung des Reaktionspartners an die Festphase meistens adsorptiv. Es wurde daher schon vorgeschlagen, die Fixierung des Reaktionspartners an die Festphase über ein Kupplungsmittel, das die Bindung vermittelt, zu bewirken. Dabei muß auch wieder darauf geachtet werden, daß die Bindung des Reaktionspartners an das Bindungsmittel nicht die spezifisch reagierende Region des Moleküls zerstört bzw. daß der Reaktionspartner so gebunden wird, daß seine reaktive Stelle von der Festphase weg dem Bindungspartner zugewandt ist. Weiterhin wird in DE-OS 25 33 701 vorgeschlagen, um eine bessere Bindung zu erzielen, die einzelnen immunologisch wirksamen Proteine zu vernetzen und dann an Polystyrolkugeln zu adsorbieren. Als weitere Möglichkeit ist in dieser Literaturstelle angegeben, gleichzeitig mit dem Protein mit immunologischen Eigenschaften ein inertes Protein zu vernetzen, so daß ein vernetztes Produkt aus inertem und aktivem Protein entsteht, das dann wiederum an Polystyrolkügelchen adsorbiert wird. Diese Art der Vernetzung führt jedoch in Abhängigkeit von den gewählten Reaktionsbedingungen zu unterschiedlichen, nicht reproduzierbaren Vernetzungen mit schwankenden Anteilen an nicht vernetztem sowie unlöslich gewordenem Protein. Durch den unterschiedlichen Vernetzungsgrad entstehen zudem Produkte mit unterschiedlichen Bindungseigenschaften. Ein ähnliches Verfahren ist in der EP-A1 122 209 beschrieben und weist auch die gleichen Nachteile auf. All diese bekannten Verfahren befriedigen somit noch nicht, führen noch zu keiner optimalen Haftung der spezifisch bindefähigen Substanz und sind für die reproduzierbare Herstellung von beschichteten Festphasen wenig geeignet.

Ziel der Erfindung war es daher, ein Verfahren zu schaffen, das die Haftung der spezifisch bindefähigen Substanz an der Festphase reproduzierbar verbessert und ein dazu geeignetes Trägermaterial zur Verfügung stellt. Da bei vielen immunologischen Verfahren unter Detergenzienzusatz gearbeitet wird, um Trübungen zu vermeiden, war es außerdem Ziel der Erfindung, die Haftung so weit zu verbessern, daß auch bei Anwesenheit von Detergenzien sich die gebundene, spezifisch bindefähige Substanz nicht ablöst.

Dieses Ziel wird erreicht durch ein Verfahren zur Herstellung einer an ein unlösliches Trägermaterial gebundenen, spezifisch bindefähigen Proteinsubstanz, insbesondere für die Verwendung in einem heterogenen Analysenverfahren, nach dem Prinzip des Immunoassay, das dadurch gekennzeichnet ist, daß man ein lösliches Protein mit einem Molekulargewicht über etwa 500.000, welches hydrophober als die spezifisch bindefähige Proteinsubstanz ist, an die spezifisch bindefähige Substanz kuppelt und dann das Konjugat aus Reaktionspartner und Protein an eine hydrophobe Festphase adsorbiert.

Die auf diese Weise festphasenfixierte spezifisch bindefähige Substanz zeigt eine verbesserte Haftung. Die Bindung ist auch gegenüber Detergenzien stabil. Bei der Erstellung von Eichkurven, die zur Auswertung bei vielen immunologischen Verfahren notwendig sind, liefert die erfindungsgemäß festphasengebundene spezifisch bindefähige Substanz steilere Eichkurven, was zu einer Erhöhung der Genauigkeit führt.

Als weiterer Vorteil des erfindungsgemäßen Verfahrens ist es möglich, die gebundene Menge genauer zu kontrollieren. Da die Haftung bedeutend besser ist als bei bisher bekannten Verfahren, ist ferner die Menge an spezifischem Protein, die eingesetzt werden muß, geringer.

Zur Auswahl von erfindungsgemäß geeigneten löslichen Proteinen muß das Molekulargewicht sowie die Hydrophobizität im Vergleich zu dem entsprechenden Wert für die spezifisch bindefähige Substanz ermittelt werden. Das Molekulargewicht wird nach den dem Fachmann bekannten Methoden ermittelt.

Ein Vergleich der Hydrophobizität zwischen löslichem Protein und spezifisch bindefähiger Substanz kann ebenfalls nach den dem Fachmann geläufigen Methoden erfolgen. Geeignete Methoden sind beispielsweise der Vergleich

- der Fluoreszenzlöschung nach Bindung an Farbstoffe (Biochem. Biophys. Acta 624, (1980), 13-20
- des Elutionsverhaltens bei der hydrophoben Chromatographie (Biochem. Biophys. Acta, 576 (1979), 269-279)
- der Oberflächenspannung (Biochem. Biophys. Acta 670 (1981), 64-73)
- der Retentionszeiten bei Hydrophobic Interaction Chromatography (HIC) (Angew. Chemie 98 (1986) 530-548, J. chromat. 296 (1984) 107-114, Anal. Biochem. 137, (1984) 464-472).

Ein Vergleich der Hydrophobizität von erfindungsgemäß geeigneten Substanzen findet sich in Sep. Sci. Technol. 14, 305-317 (1979). Danach steigt die Hydrophobizität z. B. in folgender Reihe an:

$\alpha$-2-Macroglobulin (MG 820.000)

Rinderserumalbumin/Humanserumalbumin (MG ~70.000)

Eialbumin

$\alpha_2$ HS-Glycoprotein (MG ~49.000)

$\beta_{1c}/\beta_{1A}$-Globulin

Immunglobulin (MG ~150.000)

Transferrin (MG ~90.000)

Wird also als spezifisch bindefähige Substanz ein Immunglobulin verwendet, sind beispielsweise Humanserumalbumin oder $\alpha_2$HS-Glycoprotein als lösliche Proteine im Sinne der Erfindung ohne weitere Vorbehandlung nicht geeignet.

Beide Proteine müssen hier sowohl einer Hydrophobisierung als auch einer Erhöhung des Molekulargewichts unterworfen werden. Bei Transferrin genügt in diesem Fall eine Vernetzung, bei $\alpha_2$-Macroglobulin ist eine Hydrophobisierung ausreichend.

Proteine, die zur Kupplung mit Immunglobulin als spezifisch bindefähiger Substanz ohne Vorbehandlung geeignet sind, sind beispielsweise $\beta$-Lipoproteine (MG ca. 3,2 Mio.) oder $\alpha_2$-Lipoprotein (MG ca. 5-20 Mio.).

Die Hydrophobisierung kann beispielsweise durch Anwendung von Hitze, Behandlung mit Säuren, denaturierenden Agentien und/oder chaotropen Ionen und/oder durch chemische Kupplung mit einer hydrophoben Verbindung erfolgen.

Die Erhöhung des Molekulargewichts kann beispielsweise durch Anwendung von Hitze, Behandlung mit Säuren, denaturierenden Agentien und/oder chaotropen Ionen und/oder durch Vernetzung mit einem bi- oder polyfunktionellen Proteinreagenz erfolgen.

Die Behandlung wird so lange durchgeführt, bis ein Proteinpolymeres mit einem Molekulargewicht von 500.000 oder mehr erhalten wird. Besonders bevorzugt ist es, ein Proteinpolymeres mit einem Molekulargewicht von 500.000 bis 20 Mio. zu verwenden.

Die Hydrophobisierung kann dann, wenn auch eine Vernetzung erfolgen soll, vor, während oder nach der Vernetzung, jedoch nicht in Gegenwart der spezifisch bindefähigen Substanz, vorgenommen werden.

Zur Hydrophobisierung durch Erhitzen werden üblicherweise Temperaturen von 40 bis 95°C in einem Zeitraum von 1 min bis 10 Stunden angewendet, wie beispielsweise in Biochem. Biophys. Acta 624 (1980) 13-20 beschrieben.

Als Säuren werden beispielsweise Essigsäure, Propionsäure, Milchsäure oder Salzsäure angewendet. Übliche Konzentrationen sind 1 bis 100 mmol/l Einwirkzeiten von 10 min bis 16 Stunden.

Geeignete chaotrope Ionen sind beispielsweise Thiocyanate, Jodide, Fluoride, Bromide, Perchlorate und Sulfate. Geeignete denaturierende Agentien sind beispielsweise Guanidinhydrochlorid oder Harnstoff. Üblicherweise werden hier Konzentrationen von 10 mmol/l bis 6 mol/l angewendet.

Zur Derivatisierung mit hydrophoben Verbindungen werden vorzugsweise lösliche Fettsäuren, Lipoide in nieder-oder hochmolekularer Form sowie synthetische Polymere, wie Polypropylenglykol oder lösliche Copolymere von Polystyrol eingesetzt. Die Derivatisierung erfolgt nach den dem Fachmann geläufigen Methoden.

Die Vernetzung über bi- oder polyfunktionelle Verbindungen wird mit an sich bekannten Proteinbindungsreagenzien durchgeführt. Dies sind Verbindungen, die mindestens zwei funktionelle Gruppen tragen, die gleich oder verschieden sein können und die über diese funktionellen Gruppen mit funktionellen Gruppen von Proteinen reagieren können. Bevorzugt werden Verbindungen verwendet, die aus einer Alkylkette bestehen, an deren Enden sich Succinimid-, Maleinimid- und/oder Aldehydgruppen befinden.

Das Protein wird mit der bi- oder polyfunktionellen Verbindung in an sich bekannter Weise vernetzt, indem das lösliche Protein und die bi- oder polyfunktionelle Verbindung umgesetzt werden.

Bevorzugt werden zur Hydrophobisierung und/oder Vernetzung Proteine mit einem Molekulargewicht von 10.000 bis 700.000 verwendet. Besonders bevorzugt wird Rinderserumalbumin, Lipase oder Immun-$\gamma$-Globulin.

An das Protein wird dann in an sich bekannter Weise die zu bindende spezifisch bindefähige Proteinsubstanz gekuppelt. Geeignete Kupplungsmethoden sind z. B. in Ishikawa, J. Immunoassay, 4, 209-327 (1983) beschrieben. Als spezifisch bindefähige Substanzen sind Proteine wie beispielsweise Antikörper, Antikörperfragmente, Antigene oder Haptene geeignet.

Das erhaltene Konjugat aus spezifisch bindefähiger Proteinsubstanz und Protein wird dann adsorptiv an der als Festphase dienenden Kunststoffoberfläche adsorbiert. Die adsorptive Bindung an die Festphase erfolgt über starke und schwache Wechselwirkungen, hydrophobe Kräfte, Dipol-Dipol- oder Ionen-Dipol-Interaktionen. Als hydrophobe Festphase geeignet sind Trägermaterialien mit einer Oberflächenspannung, die kleiner als die Oberflächenspannung des hydrophoben löslichen Proteins ist, d.h. hydrophober sind als Protein. Bevorzugt werden Trägermaterialien mit einer Oberflächenspannung < 40 erg/cm$^2$ eingesetzt. Besonders geeignet sind Polystyrol, Polymethacrylat, Teflon, Polyamid, Copolymere aus Styrol und Acrylnitril, Glas und Celluloseprodukte. Sie können in beliebiger Form vorliegen, z.B. als Film, Platte, Pulver, Körnchen oder Faservlies, bevorzugt als Glasfaservlies oder Vlies aus Cellulose-/Celluloseesterfaser und Polymerisatfaser.

Hydrophobisierte Proteine zeigen eine besonders gute adsorptive Bindung. Möglicherweise werden durch die Hydrophobisierung intramolekulare Brückenbindungen des Proteins geöffnet, so daß hydrophobe Teile des Proteins an die Oberfläche gelangen und dort besser an der hydrophoben Kunststoffoberfläche haften, als die hydrophilen Teile, die im nicht hydrophobisierten Protein in erster Linie an der Oberfläche zu finden sind.

Das erfindungsgemäße Verfahren eignet sich für die Bestimmung einer spezifisch bindefähigen Substanz. Als Substanzpaare, von denen ein Reaktionspartner an die Festphase gebunden vorliegt, sind beispielsweise geeignet Antigen-Antikörper; Hapten-Antikörper und andere spezifisch miteinander zur Bindung befähigte Proteine, wie insbesondere das System Streptavidin bzw. Avidin und Biotin, welches bevorzugt wird.

Bevor das Konjugat aus Protein und spezifisch bindefähiger Substanz an die hydrophobe Festphase adsorbiert wird, ist es auch möglich, die Festphase physikalisch oder chemisch vorzubehandeln. So kann beispielsweise eine Kunststoffoberfläche vorgequollen werden oder in anderer an sich bekannter Weise aktiviert werden.

Das erfindungsgemäße Trägermaterial zur Verwendung bei Festphasen-Immunoassays ist dadurch gekennzeichnet, daß es aus einer hydrophoben Festphase besteht, an die ein Protein mit einem Molekulargewicht von über etwa 500.000 adsorbiert ist, an das eine spezifisch bindefähige Substanz gebunden ist.

Dieses Trägermaterial eignet sich ausgezeichnet zur Verwendung für Festphasen-Immunoassays, da die spezifisch bindefähige Substanz sehr gut haftet und auch bei Zusatz von Detergenzien nicht desorbiert.

Das Trägermaterial liegt beispielsweise in Form von Röhrchen, Mikrotiterplatten oder Kugeln vor, die beschichtet sind mit einem vernetzten Protein, an das eine spezifisch bindefähige Substanz gebunden ist.

An die Festphase ist ein Konjugat, bestehend aus einem vernetzten Protein und einer spezifisch bindefähigen Substanz adsorbiert. Das Protein ist bevorzugt Rinderserumalbumin, Lipase oder ein Immun-$\gamma$-Globulin, das in der angegebenen Weise vernetzt wurde.

Erfindungsgemäß wird ein Verfahren und ein Trägermaterial zur Verfügung gestellt, um eine spezifisch bindefähige Substanz mit guter Haftung und dauerhaft an eine hydrophobe Festphase zu fixieren. Die Haftung konnte so weit verbessert werden, daß auch ein Zusatz von Detergenzien nicht zu einer Ablösung der Substanz führt. Das erfindungsgemäße Verfahren ist einfach durchzuführen.

Die Erfindung wird durch Beispiele in Verbindung mit der Zeichnung erläutert. In dieser stellen dar:

Fig. 1     eine Eichkurve für eine TSH-Bestimmung unter Verwendung von Fab<TSH> unvernetzt (Kurve 1),

Fab<TSH> vernetzt (Kurve 2),

Konjugat Fab<TSH>/$\beta$-Gal (Kurve 3),

Konjugat Fab<TSH>/Thermo-RSA (Kurve 4) in Luran ®-Röhrchen

Fig. 2     eine Eichkurve für eine TSH-Bestimmung unter Verwendung von immobilisiertem Streptavidin und biotinyliertem Ak<TSH>

Steptavidin unvernetzt (Kurve 1),

Streptavidin vernetzt (Kurve 2),

Streptavidin/$\beta$-Gal-Konjugat (Kurve 3),

Streptavidin/RSA-Konjugat (Kurve 4),
Streptavidin/Thermo-RSA-Konjugat (Kurve 5),
Streptavidin/Thermo-RSA-Konjugat (Kurve 6).
Kurve 1 bis 5 jeweils Luran ®-Röhrchen als Festphase, Kurve 6 Polystyrol-Röhrchen als Festphase

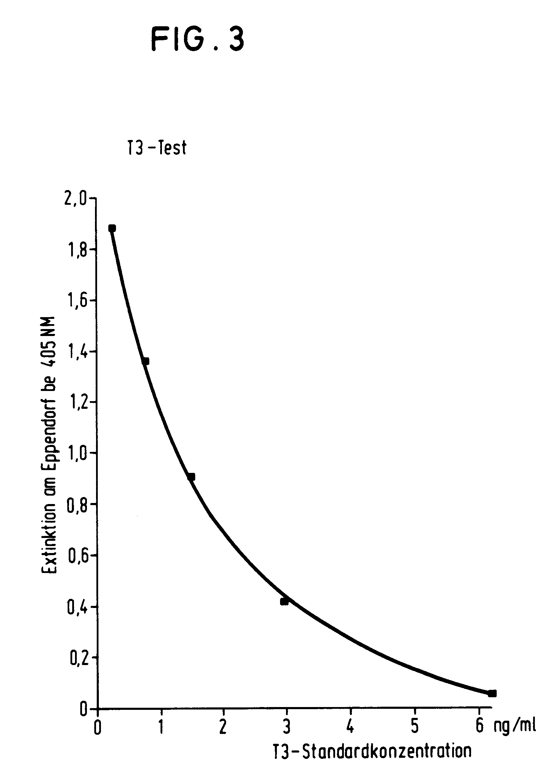

Fig. 3      eine Eichkurve für einen T3-Test

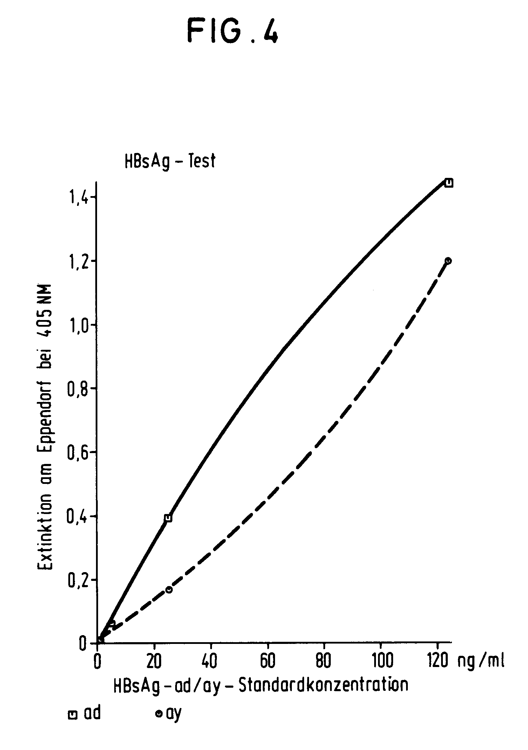

Fig. 4      eine Eichkurve für einen HBs AG-Test

**Beispiel 1**

Bindung von Fab-Fragmenten eines monoklonalen Antikörpers gegen TSH an Polystyrolröhrchen

a) Herstellung von Fab-Fragmente (Fab<TSH>)

Monoklonale Antikörper (MAK<TSH>) werden nach der von Galfre und Millstein beschriebenen Methode (Meth. in Enzymology 73 (1981)) gewonnen. Zur weiteren Reinigung wird die Ascitesflüssigkeit einer Ammoniumsulfatfällung und einer Passage über DEAE-Cellulose® unterworfen.

Anschließend wird nach Biochem. Y. 73 (1959) 119-126 eine Papainspaltung durchgeführt. Die hierbei entstandenen Fab-Fragmente werden von den nichtverdauten IgG-Molekülen und den Fc-Fragmenten mittels Gelfiltration über Sephadex G100® und Ionenaustauscherchromatographie über DEAE-Cellulose® nach Meth. in Enzymology 73 (1981) 418-459 abgetrennt.

b) Vernetzung der Fab-Fragmente ohne Proteinzusatz (Vergleich)

50 mg Fab-Fragmente werden in 2 ml 0,05 mol/l Kaliumphosphatpuffer (pH 7,5) gelöst und 0,4 ml Disuccinimidylsuberat (Hersteller Pierce), gelöst in Dioxan (7,4 mg/ml), unter Rühren zugegeben. Nach 2 Stunden Inkubation bei 25°C wird durch Zugabe von 0,2 ml 0,1 mol/l Lysinhydrochlorid die Reaktion abgebrochen. Der Reaktionsansatz wird mit 0,2 ml Kaliumphosphatpuffer (s. o.) verdünnt und zentrifugiert. Der Überstand wird über eine Ultrogel® AcA 202-Säule (LKB, Gräfelfing/BRD) entsalzt, wobei 11,3 ml mit 45 mg Protein erhalten werden. Ein Teil dieses Präparates wird an einer Superose ®-6-Säule (Deutsche Pharmacia GmbH) bei 0,5 ml/min Durchflußrate in 0,05 mol/l Kaliumphosphatpuffer pH 7,0 fraktioniert und die Fraktion mit Molekulargewicht ca. 500.000 - 5 Mio. weiterverwendet.

c) Vernetzung der Fab-Fragmente mit unbehandeltem γ-Globulin (Vergleich)

Fab-Fragmente und γ-Globulin aus Rind (Serva, Heidelberg/BRD) werden im Massenverhältnis 1:1 vermischt. Die Vernetzung wird, wie unter b) beschrieben, durchgeführt.

d) Bindung von Fab-Fragmenten an vorvernetztes γ-Globulin (erfindungsgemäß)

1,25 g γ-Globulin werden in 10 ml 0,05 mol/l Kaliumphosphatpuffer, pH 7,8, gelöst und in einer Sorvall-Kühlzentrifuge 10 min bei 5.000 UpM blankzentrifugiert. Zu dieser Lösung werden 1,75 ml Disuccinimidylsuberat zugesetzt und mit 2,5 ml Wasser verdünnt. Nach 4 Stunden Rühren bei 25°C werden 10 ml 0,1 mol/l Lysin zugesetzt und der pH-Wert auf 6,8 eingestellt und zentrifugiert. Der Überstand wird an einer präparativen Gelfiltrationssäule (TSK 3000, LKB Gräfelfing/BRD) abgetrennt, über Ultrafiltration eingeengt und bei 4°C aufbewahrt.

50 mg dieses vernetzten γ-Globulins werden in 5 ml 0,05 mol/l Kaliumphosphatpuffer gelöst und der pH-Wert durch Zugabe von festem Natriumcarbonat auf 9,5 eingestellt. Dann werden 50 mg N-Acetylhomocysteinthiolacton (Serva, Heidelberg/BRD) zugegeben und 5 Stunden bei 25°C unter Stickstoffbegasung gerührt. Der Ansatz wird anschließend entsalzt über eine Ultrogel ® AcA 202-Säule in einem Puffer aus 0,1 mol/l Kaliumphosphat (pH 7,0) 0,001 mol/l Magnesiumchlorid und 0,05 mol/l Natriumchlorid.

Nach a) hergestellte Fab-Fragmente (10 mg in 1 ml 0,01 mol/1 Kaliumphosphatpuffer pH 7,0) werden mit 0,002 ml Maleinimidohexanoyl-N-hydroxysuccinimidester (Boehringer Mannheim GmbH) in DMSO (33 mg/ml) 2 Stunden bei 25°C aktiviert, anschließend wird zentrifugiert und über eine AcA 202-Säule entsalzt.

Diese Fragmente werden anschließend mit dem γ-Globulin vereinigt (Massenverhältnis der Proteine 1:1) und 1 Stunde bei 25°C und pH 7,0 inkubiert. Anschließend wird gegen entsalztes Wasser über Nacht bei 4°C und einer Proteinkonzentration von 2,5 mg/ml dialysiert.

Dieses Konjugat kann direkt zur Adsorption an eine Festphase verwendet werden.

e) Herstellung von Fab-Fragmenten gekoppelt an Thermo-RSA (Rinderserumalbumin; erfindungsgemäß)

Herstellung von Thermo-RSA (Rinderserumalbumin): 1 g RSA-I werden in 100 ml 50 mmol/l Kaliumphosphat (pH 7,0) gelöst, auf 70°C erhitzt und 4 Stunden unter leichtem Rühren bei dieser Temperatur gehalten. Die Lösung wird abgekühlt, filtriert und in einer Ultrafiltrationszelle (Ausschlußgrenze: 30.000 Dalton) auf eine Konzentration von 50 mg/ml eingestellt. Anschließend wird gegen das 30fache Volumen an bidest. $H_2O$ dialysiert und anschließend lyophilisiert. Das Produkt hat ein Molekulargewicht von ca.

700.000.

Vor Kupplung an die Fab-Fragmente wird Thermo-RSA aktiviert. Hierzu werden 68 mg Thermo-RSA in 2 ml 0,1 mol/l Kaliumphosphat (pH 7,8) gelöst und langsam mit einer Lösung von 3,8 mg S-Acetylmercaptobernsteinsäureanhydrid (SAMBA) versetzt. Nach 3 Stunden Reaktionszeit wird gegen 2 l 50 mmol/l Kaliumphosphat (pH 6,5) bei 4°C dialysiert. Dieses Thermo-RSA wird mit den nach d) aktivierten Fab-Fragmenten im Massenverhältnis 1:1 1 Stunde bei 25°C und pH 7,0 inkubiert, anschließend gegen entsalztes Wasser über Nacht bei 4°C und einer Proteinkonzentration von 2,5 mg/ml dialysiert.

Dieses Produkt wird direkt zur Beschichtung eingesetzt.

f) Herstellung von an β-Galactosidase gekuppelten Fab-Fragmenten (erfindungsgemäß)

Fab-Fragmente werden, wie in d) beschrieben, aktiviert und an die SH-Gruppen von β-Galactosidase nach J. Immunoassay 4 (1983) 209-327 gekuppelt. Die eingesetzte β-Galactosidase hat hierbei ein Molekulargewicht von 500.000 bis 2 Mio.

Zu einem weiteren Versuch wurde vernetzte β-Galactosidase (MG ~ 5 Mio.) eingesetzt.

g) Beladung von Polystyrolröhrchen mit Fab-Fragmenten bzw. deren Konjugaten

50 mg eines Lyophilisats des Fab-Fragments bzw. des Konjugats werden in 10 ml bidest. Wasser gelöst. 1 ml dieser Lösung wird in 1.000 ml eines Beladungspuffers aus 5,25 g/l Natriumdihydrogenphosphat und 1 g/l Natriumazid verdünnt und 30 min bei Raumtemperatur gerührt.

In jedes Röhrchen Polystyrol bzw. Luran ® (Hersteller BASF) werden 1,5 ml der Lösung gefüllt und über Nacht (ca. 22 Stunden) beladen. Danach werden die Röhrchen vollständig entleert und der nachstehend beschriebene Funktionstest durchgeführt.

h) Funktionstest über TSH-Bestimmung

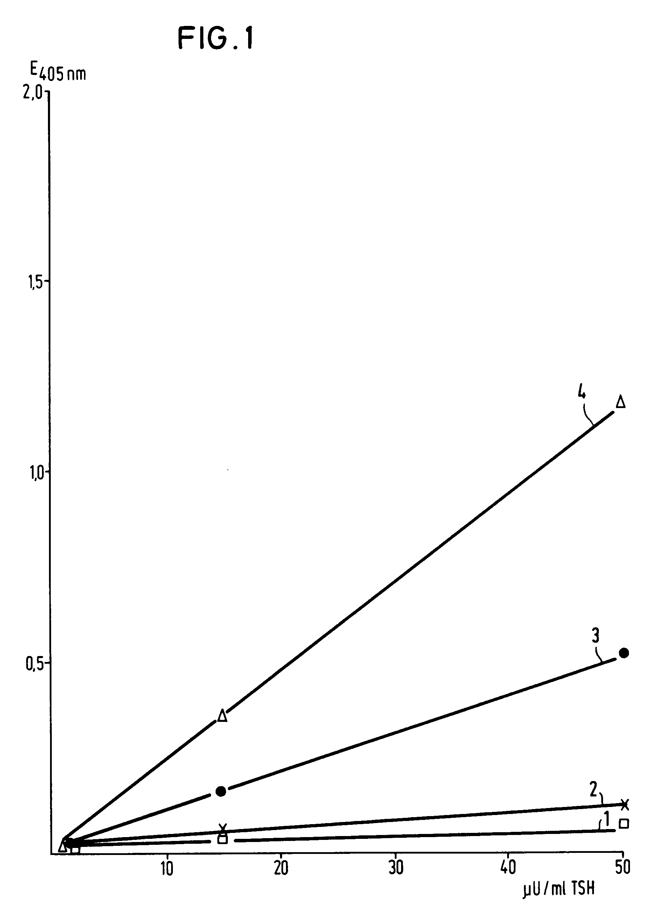

Die nach g beschichteten Polystyrol- bzw. Luran ® -Röhrchen werden in einem TSH-Bestimmungsreagenz analog TSH-Enzymun ® -Test (Boehringer Mannheim GmbH, Best.-Nr. 736 082) eingesetzt und eine Eichkurve entsprechend der Testvorschrift vermessen. Hierbei ergeben sich die in Tabelle 1 bzw. Fig. 1 dargestellten Meßwerte.

Es zeigt sich (Fig. 1), daß mit Fab-Fragmenten, welche ohne Proteinzusatz immobilisiert wurden, nur eine sehr flache Eichkurve erhalten werden kann (Kurven 1 und 2). Durch Kupplung an Proteine mit einem Molekulargewicht unter 500.000 und geringer Hydrophobizität ist die Eichkurve steiler (Kurve 3). Allerdings können immer noch keine befriedigenden Ergebnisse erreicht werden. Mit den erfindungsgemäß hergestellten Konjugaten kann dagegen eine ausreichend steile Eichkurve erreicht werden (Kurve 4).

<u>T a b e l l e   1</u>

Standardwerte für verschiedene Fab⟨TSH⟩-Konjugate

| Konjugat* | Hydropho-bizität des Proteins** | Molekular-gew. des Proteins | Meßsignal von TSH-Standards in mE/ml | |
|---|---|---|---|---|
| | | | 0,1 µU TSH[1] | 12,5 µU TSH[2] |
| Fab⟨TSH⟩ | – | – | 102 | 107 |
| Fab⟨TSH⟩ vernetzt ohne Protein (Bsp. 1b) | – | – | 126 | 134 |
| Fab⟨TSH⟩/ß–Gal (Bsp. 1f) | 39,6 | 500.000 | 110 | 260 |
| Fab⟨TSH⟩/ß–Gal vernetzt (Bsp. 1f) | 40,2 | 5 Mio | 110 | 260 |
| Fab⟨TSH⟩/ γ-Globulin (Bsp. 1c) | 32,0 | 150.000 | 117 | 253 |
| Fab⟨TSH⟩/ γ-Globulin (Bsp. 1d) | nicht eluier-bar | >5 Mio | 75 | 480 |

\* Gesamtproteinmenge bei Adsorption: 1,5 ml mit 5 µg/ml Protein pro Röhrchen
\*\* $T_p$ /min/ vgl. Beispiel 3
1) niedrigster Wert einer Messkurve (sollte mögl. niedrig sein)
2) höchster Werkt einer Messkurve (sollte möglichst hoch sein)

**Beispiel 2**

Immobilisierung von Streptavidin

a) Herstellung von vernetztem Streptavidin
Streptavidin (Hersteller: Boehringer Mannheim GmbH) wird analog Beispiel 1b vernetzt.
b) Herstellung von Streptavidin gebunden an RSA bzw. β-Galactosidase
Die Bindung an nichtvernetztes RSA bzw. β-Galactosidase erfolgt analog Beispiel 1f.
c) Herstellung von Streptavidin gekuppelt an Thermo-RSA
Aktivierung von Streptavidin:

60 mg Streptavidin werden in 6 ml 50 mmol/l Kaliumphosphat/100 mmol/l Natriumchlorid (pH 7,0) gelöst und bei 4°C kaltgestellt. 6,16 mg Maleinimidohexanoyl-N-hydroxysuccinimidester werden in 620 $\mu$l Dioxan gelöst und in die Streptavidinlösung eingerührt. Nach einer Reaktionszeit von 2 Stunden bei 4°C wird gegen 2mal 1l 50 mmol/l Kaliumphosphat/100 mmol/l Natriumchlorid (pH 5) bei 4°C dialysiert.

Herstellung eines Konjugats aus Streptavidin und Thermo-RSA:

66 mg Streptavidin in 10 ml 50 mmol/l Kaliumphosphat pH 5,0 und 100 mmol/l Natriumchlorid gelöst und 72 mg aktiviertes Thermo-RSA-SAMBA (Herstellung nach Beispiel 1e)) in 5 ml 50 mmol/l Kaliumphosphat/100 mmol/l Natriumchlorid pH 6,5 werden zugegeben. Nach Vermischen werden 50 $\mu$l 1 mol/l Hydroxylamin pH 7,0 zugegeben, um die Reaktion zu stoppen. Nach 3 Stunden wird das Reaktionsprodukt über Gelchromatographie (Superose ® 6, 50 mmol/l Kaliumphosphat/100 mmol/l Natriumchlorid pH 7,5) aufgereinigt. Es wird ein Konjugat mit einem Molekulargewicht zwischen 1 und 5 Mio. erhalten.

d) Beladung von Polystyrol- bzw. Luran ® -Röhrchen mit Streptavidin bzw. Streptavidinkonjugat

Die Beladung erfolgt, wie in Beispiel 1 g) beschrieben.

e) Messung von Standardwerten über eine TSH-Bestimmung

Die nach d) beladenen Röhrchen werden in einem TSH-Enzymun ® -Test-Reagenz (4fache Konjugataktivität) eingesetzt. In Abweichung von der dort beschriebenen Prozedur wird jedoch anstelle des immobilisierten Antikörpers ein biotinylierter MAK<TSH> verwendet. Die Herstellung dieses biotinylierten MAK's erfolgt nach JACS 100 (1978) 3585-3590. Der Antikörper wird im Test in einer Konzentration von 400 ng pro Teströhrchen zusammen mit den anderen Reagenzien eingesetzt.

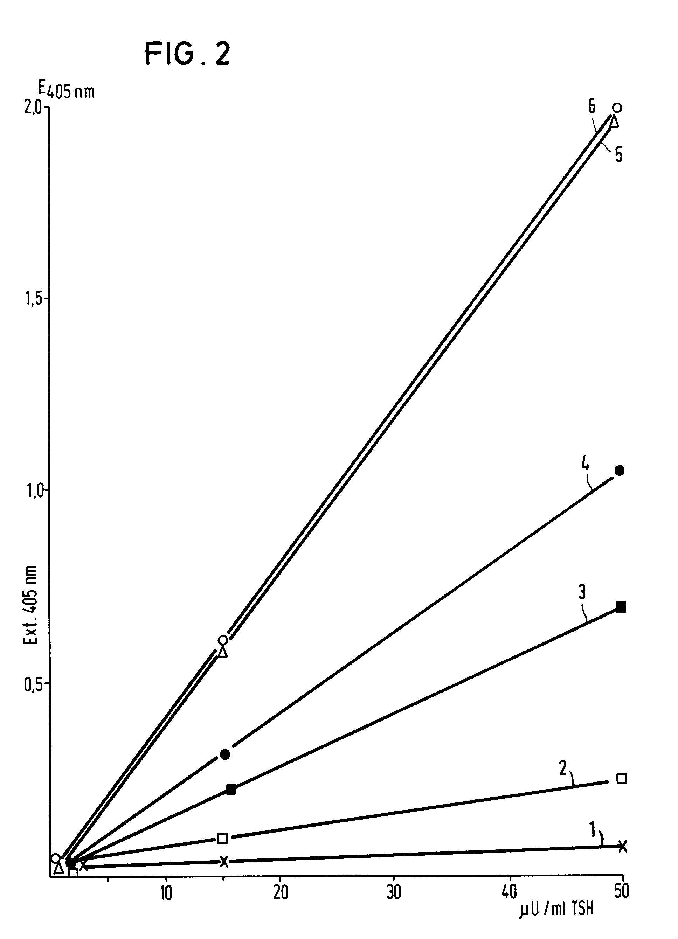

Die Ergebnisse zeigt Fig. 2. Daraus ist zu ersehen, daß mit zunehmendem Molekulargewicht und steigender Hydrophobizität die Steigung der Eichkurve und damit die erzielbare Genauigkeit zunimmt.

**Beispiel 3**

Mit den erfindungsgemäß beladenen Röhrchen wurde ein T3-Test durchgeführt. Dazu wurden 200 $\mu$l Probe bzw. Standardlösung zusammen mit 500 $\mu$l eines polyklonalen Antikörperkonjugates gegen T3, das mit POD markiert war, in einem Röhrchen, das mit Streptavidin gekuppelt an Thermo-RSA beschichtet war, vorinkubiert. Nach 5 Minuten werden 400 ng in bekannter Weise biotinyliertes polymerisiertes T3 in 500 $\mu$l Puffer 30 Minuten lang inkubiert. Als Puffer wurde eine Lösung von Natriumhydrogenphosphat mit einem pH von 8,65, die 0,20 % RSA und 0,04 % 8-Anilino-1-Naphthalinsulfonsäure (ANS) enthielt, verwendet. Nach der Inkubation wurde dreimal gewaschen und anschließend 1 ml ABTS-Substratlösung zugegeben. Nach weiteren 30 Minuten Inkubation wurde dann bei 405 nm am Photometer gemessen. Die Meßwerte sind der in Fig. 3 dargestellten Kurve zu entnehmen.

**Beispiel 4**

Mit den erfindungsgemäß beschichteten Röhrchen wurde ein HBsAg-Test durchgeführt. Dazu wurden in ein gemäß Beispiel 1g) beschichtetes Röhrchen gleichzeitig 400 ng biotinylierter monoklonaler Antikörper gegen HBs-Antigen und 50 mU desselben Antikörpers, die mit POD markiert waren, gelöst in 1 ml Puffer derselben Zusammensetzung, wie im Beispiel 3 beschreiben, gegeben, zusammen mit 200 $\mu$l Standardlösung. Als Standardlösung wurde einmal aufgereinigtes HBsAg Subtype ay und einmal aufgereinigtes HBsAg Subtype ad gegeben. Es wurde dann vier Stunden bei Raumtemperatur inkubiert. Nach dreimaligem Waschen der Röhrchen wurde 1 ml ABTS-Substratlösung zugegeben. Nach 20 Minuten war die Reaktion im wesentlichen beendet und die Extinktion wurde bei 405 nm am Photometer gemessen. Die Meßwerte sind der Fig. 4 zu entnehmen, wobei die durchgezogene Kurve die Werte für HBsAg Subtype ad wiedergibt und die gestrichelte Kurve die Werte für HBsAg Subtype ay.

**Beispiel 5**

Es wurde die Ablösung der erfindungsgemäß beschichteten Röhrchen mit nach bekannten Verfahren beschichteten Röhrchen verglichen. Dazu wurden einmal Röhrchen mit 1,5 ml einer Streptavidin-Thermo RSA-Lösung (4 $\mu$g/ml) in 40 mM Natriumhydrogenphosphatpuffer, pH 7,4, bei 20°C 18 bis 24 Stunden beladen. Nach Aussaugen der Röhrchen erfolgte eine Nachbeladung mit 1,8 ml 2%iger Saccharoselösung, die 0,9 % Natriumchlorid und 0,3 % RSA enthielt. Die Nachbeladung erfolgte 30 Minuten lang bei 20°C. Anschließend werden die Röhrchen 24 Stunden lang bei 20°C und 40 % relativer Feuchte getrocknet. Diese Röhrchen sind für die Durchführung von Tests einsatzbereit. Weiterhin wurden in an sich bekannter Weise Röhrchen mit reinem Streptavidin beladen. Mit diesen Röhrchen wurde das Ablösungsverhalten bei Einwirkung von Detergenzien getestet. Die Ergebnisse sind der folgenden Tabelle zu entnehmen.

T a b e l l e  2

| Beladung | Röhrchen Material | % Ablösung 1 % Pluronic F 68 (30 min, RT) | % Ablösung 0,5 % Triton (30 min, RT) | % Ablösung Funktionstest (30 min, 20°C) | % Ablösung Funktionstest (30 min, 30°C) |
|---|---|---|---|---|---|
| Streptavidin (Vergleich) | Luran | 13,9 | 42 | 17,8 | 28 |
| | Polystyrol | 11 | 25 | 9,6 | 11,6 |
| Thermo-RSA-Streptavidin (Erfindung) | Luran | 2,1 | 8 | 3,1 | 11,6 |
| | Polystyrol | 1,5 | 2,1 | 1,6 | 1,7 |

Die Ermittlung der prozentualen Ablösung erfolgt nach den dem Fachmann geläufigen Methoden, z.B. über eine $^{125}$J-Markierung von Streptavidin und Streptavidin-Thermo-RSA oder eine enzymatische Bestimmung.

9

Zur enzymatischen Bestimmung der prozentualen Ablösung werden die beschichteten Röhrchen mit 50 mmol/l Kaliumphosphatpuffer, pH 7,0, dem Detergens gemäß Tabelle II zugesetzt wurde, bei den Bedingungen gemäß Tabelle II inkubiert.

Anschließend wird 1 Stunde bei Raumtemperatur inkubiert, mit o.g. Puffer gewaschen und mit einem Konjugat aus Biotin-POD (200 mU/ml POD Aktivität), 1 Std bei Raumtemperatur inkubiert, gewaschen und 2 ml ABTS®-Lösung (Beispiel 7) zugegeben. Nach 1 Std. Substratreaktion wird die Extinktion bei 405 nm bestimmt und hieraus die prozentuale Ablösung von Streptavidin bzw. Strepavidin-Thermo-RSA über eine Standard-Eichkurve ermittelt.

**Beispiel 6**

Bestimmung der Hydrophobizität von Proteinen mit Hydrophobic Interaction Chromatography (HIC)

Die Hydrophobizität von verschiedenen Verbindungen wurden mit einem Flüssigkeitschromatograph (Hewlett Packard 1090 LUSI) untersucht. Vorsäule war eine BioRad-Biogel ® TSK-Phenyl-5PW (L 5 mm × ID 4,6 mm), Säule: BioRad-Biogel ® TSK-Phenyl-5PW (L 75 mm × ID 7,5 mm, 10 $\mu$m, 1.000 Å). Als Detektor wurde ein Hitachi F 1.000-Fluorimeter verwendet.

Eluenten/Gradient (Tabelle 3)

a) 1,5 mol/l $(NH_4)_2SO_4$-Lösung in 1/100 mol/l $KH_2PO_4$-Puffer, pH 6,8
b) 1/100 mol/l $KH_2PO_4$-Puffer, pH 6,8

### T a b e l l e   3

| % A | % B | t/min | |
|---|---|---|---|
| 100 | 0 | 0 | |
| 100 | 0 | 5 | |
| 0 | 100 | 30 | Flow = 0,5 min |
| 0 | 100 | 5 | |
| 100 | 0 | 5 | |
| 100 | 0 | 5 | |

Arbeitstemperatur:
Kühlraum, +7°C
Probenvorbereitung:
Die Proben wurden unverdünnt eingesetzt. Das Probevolumen betrug 10 $\mu$l.

Die zu untersuchenden Verbindungen wurden in einer Konzentration von 0,2 bis 1,4 mg/ml in 10 mmol/l Kaliumphosphatpuffer pH 6,8 gelöst.

In Tabelle 4 sind die Retentionszeiten für verschiedene Proteine bzw. spezifisch bindefähige Substanzen zusammengestellt. Je länger die Retentionszeit, desto größer die Hydrophobizität.

## T a b e l l e   4

| Protein | Retentionszeit $t_p$ (min) |
|---|---|
| Fab TSH | 41,5 |
| RSA | 23,2 |
| $\gamma$-Globulin | 32,0 |
| ß-Galactosidase | 39,6 |
| ß-Galactosidase vernetzt | 40,2 |
| Streptavidin | 38,3 |
| Thermo-RSA (Bsp. 1e) | nicht eluierbar |
| $\gamma$-Globulin, vernetzt (Bsp. 1a) | nicht eluierbar |

Besonders geeignet sind die unter diesen Bedingungen nicht eluierbaren Proteine, die bevorzugt verwendet werden. Besonders bevorzugt wird Thermo-RSA.

Besonders geeignet sind die unter diesen Bedingungen nicht eluierbaren Proteine, die bevorzugt verwendet werden. Besonders bevorzugt wird Thermo-RSA.

**Beispiel 7**

Adsorption von Thermo-RSA-Streptavidin an Glasfaservlies

Ein Glasfaservlies (6 × 6 mm) wird im Aufsaugvolumen (ca. 30 µl) mit einer Lösung von 30 µg/ml Thermo-RSA-Streptavidin (Herstellung Beispiel 2c) in 50 mMol/l Kaliumphosphatpuffer, pH 7,0 getränkt und bei 50°C im Umluftschrank getrocknet.

Zur Bestimmung der Biotin-Bindekapazität wird der Streifen mit 30 µl eines Reagenzes, bestehend aus Konjugat aus Peroxidase (POD) und Biotin mit einer POD-Aktivität von 50 mU/ml
Biotinstandard mit den Konzentrationen 0,5, 10, 20, 30, 40, 50, 100, 200, 1000 ng/ml Biotin
getränkt und 2 Minuten inkubiert. Anschließend wird einmal mit einem Überschuß an 50 mMol/l Kaliumphosphatpuffer, pH 7,0 2 %o-Tween®-20 gewaschen und anschließend in 2 ml ABTS-Lösung
100 mMol/l Citratpuffer, pH 4,4
3,2 mMol/l Perborat
1,9 Mol/l ABTS® (2,2'-Azino-di[3-ethyl-benzthiazolinsulfonsäure(6)]-diammoniumsalz)
eingebracht und 15 Minuten in Bewegung gehalten. Nach 1 Stunde Substratreaktion wird die Extinktion bei 405 nm bestimmt und hieraus die Biotinbindekapazität über den halbmaximalen Signalabfall ermittelt.

**Patentansprüche**

1. Verfahren zur Herstellung einer an ein unlösliches Trägermaterial gebundenen, spezifisch bindefähigen Proteinsubstanz, insbesondere für die Verwendung in einem heterogenen Analysenverfahren, nach dem Prinzip des Immunoassay, **dadurch gekennzeichnet,** daß man ein lösliches Protein mit einem Molekulargewicht über etwa 500.000, welches hydrophober als die spezifisch bindefähige Proteinsubstanz ist, an die spezifisch bindefähige Substanz kuppelt und dann das Konjugat aus Reaktionspartner und Protein an eine hydrophobe Festphase adsorbiert.

**2.** Verfahren nach Anspruch 1, **dadurch gekennzeichnet,** daß man als lösliches Protein ein Protein mit einem Molekulargewicht von 500.000 bis 20 Mio verwendet.

**3.** Verfahren nach Anspruch 2, **dadurch gekennzeichnet,** daß man das lösliche Protein aus einem Protein mit einem Molekulargewicht von 10.000 bis 700.000 durch Erhöhung des Molekulargewichts über 500.000 bis 20 Mio herstellt.

**4.** Verfahren nach einem der Ansprüche 1 bis 3, **dadurch gekennzeichnet,** daß man das Protein vor Kupplung mit einem bi-oder polyfunktionellen Proteinreagenz vernetzt, bis das gewünschte Molekulargewicht erreicht ist.

**5.** Verfahren nach einem der vorhergehenden Ansprüche, **dadurch gekennzeichnet,** daß man ein hydrophobisiertes Protein verwendet.

**6.** Verfahren nach Anspruch 5, **dadurch gekennzeichnet,** daß das Protein vor Kupplung durch Anwendung von Hitze, Behandlung mit Säuren, denaturierenden Agentien oder chaotropen Ionen und/oder durch chemische Kupplung mit einer hydrophoben Verbindung hydrophobisiert wird.

**7.** Verfahren nach Anspruch 4, **dadurch gekennzeichnet,** daß man als bifunktionelle Verbindung Disuccinimidylsuberat verwendet.

**8.** Verfahren nach einem der Ansprüche 3 bis 7, **dadurch gekennzeichnet,** daß man als Protein Rinderserumalbumin, Lipase und/oder Immun-γ-Globuline verwendet.

**9.** Trägermaterial zur Verwendung bei Festphasen-Immunoassays, erhältlich nach einem der Ansprüche 1 bis 8, **dadurch gekennzeichnet,** daß es aus einer hydrophoben Festphase besteht, an die ein Konjugat aus einem hydrophoben Protein mit einem Molekulargewicht von über etwa 500.000 und einer spezifisch bindefähigen Proteinsubstanz adsorbiert ist.

**10.** Trägermaterial nach Anspruch 9, **dadurch gekennzeichnet,** daß die hydrophobe Festphase aus Polystyrol, Polymethacrylat, Polyamid, Teflon® oder aus einem Copolymeren von Styrol und Acrylnitril besteht.

**11.** Trägermaterial nach Anspruch 9 oder 10, **dadurch gekennzeichnet,** daß das Protein hydrophobisiertes Rinderserumalbumin (Thermo-RSA), hydrophobisierte Lipase oder hydrophobisiertes Immun-γ-Globulin ist.

**12.** Trägermaterial nach einem der Ansprüche 9 bis 11, **dadurch gekennzeichnet,** daß die spezifisch bindefähige Substanz ein Antigen oder ein Antikörper ist.

**13.** Trägermaterial nach einem der Ansprüche 9 bis 11, **dadurch gekennzeichnet,** daß die spezifisch bindefähige Substanz Streptavidin oder Avidin ist.

**14.** Trägermaterial nach einem der Ansprüche 9 bis 13, **dadurch gekennzeichnet,** daß der Träger ein Glasfaservlies oder ein Vlies aus Cellulose-/Celluloseesterfaser und Polymerisatfaser ist.

**Claims**

**1.** Process for the preparation of a specifically bindable protein substance bound to an insoluble carrier material, especially for use in a heterogeneous analysis process, according to the immunoassay principle, characterised in that one couples a soluble protein with a molecular weight above about 500,000, which is more hydrophobic than the specifically bindable substance, to the specifically bindable substance and then adsorbs the conjugate of reaction component and protein on a hydrophobic solid phase.

**2.** Process according to claim 1, characterised in that, as soluble protein, one uses a protein with a molecular weight of 500,000 to 20 mio.

**3.** Process according to claim 2, characterised in that one prepares the soluble protein from a protein with a molecular weight of 10,000 to 70,000 by increasing the molecular weight to above 500,000 to 20 mio.

**4.** Process according to one of claims 1 to 3, characterised in that, before coupling, one cross-links the protein with a bi- or polyfunctional protein reagent until the desired molecular weight is achieved.

**5.** Process according to one of the preceding claims, characterised in that one uses a hydrophobed protein.

**6.** Process according to claim 5, characterised in that, before coupling, the protein is hydrophobed by the use of heat, treatment with acids, denaturing agents or chaotropic ions and/or by chemical coupling with a hydrophobic compound.

**7.** Process according to claim 4, characterised in that one uses disuccintmidyl suberate as bifunctional compound.

**8.** Process according to one of claims 3 to 7, characterised in that one uses bovine serum albumin, lipase and/or immune $\gamma$-globulins as protein.

**9.** Carrier material for use in solid phase immunoassays obtainable according to one of claims 1 to 8, characterised in that it consists of a hydrophobic solid phase on which is adsorbed a conjugate of a hydrophobic protein with a molecular weight above about 500,000 and of a specifically bindable protein substance.

**10.** Carrier material according to claim 9, characterised in that the hydrophobic solid phase consists of polystyrene, polymethacrylate, polyamide, Teflon® or a co-polymer of styrene and acrylonitrile.

**11.** Carrier material according to claim 9 or 10, characterised in that the protein is hydrophobed bovine serum albumin (thermo-BSA), hydrophobed lipase or hydrophobed immune $\gamma$-globulin.

**12.** Carrier material according to one of claims 9 to 11, characterised in that the specifically bindable substance is an antigen or an antibody.

**13.** Carrier material according to one of claims 9 to 11, characterised in that the specifically bindable substance is streptavidin or avidin.

**14.** Carrier material according to one of claims 9 to 13, characterised in that the carrier is a glass fibre fleece or a fleece of cellulose/cellulose ester fibres and polymer fibres.

**Revendications**

**1.** Procédé de préparation d'une substance protéique capable de liaison spécifique, liée à un matériau support insoluble, en particulier en vue d'une utilisation dans un procédé d'analyse hétérogène selon le principe du dosage immunologique, caractérisé en ce que l'on couple à la substance capable de liaison spécifique une protéine soluble d'un poids moléculaire supérieur à environ 500000 qui est plus hydrophobe que la substance protéique capable de liaison spécifique puis l'on adsorbe sur une phase solide hydrophobe le conjugué constitué par le partenaire réactionnel et la protéine.

**2.** Procédé selon la revendication 1, caractérisé en ce que l'on utilise comme protéine soluble une protéine d'un poids moléculaire de 500000 à 20 millions.

**3.** Procédé selon la revendication 2, caractérisé en ce que l'on prépare la protéine soluble à partir d'une protéine d'un poids moléculaire de 10000 à 700000 par augmentation du poids moléculaire à une valeur supérieure à 500000 et atteignant 20 millions.

**4.** Procédé selon l'une des revendications 1 à 3, caractérisé en ce que l'on réticule la protéine, avant le couplage, avec un réactif protéique di- ou polyfonctionnel jusqu'à ce que le poids moléculaire souhaité soit atteint.

**5.** Procédé selon l'une des revendications précédentes, caractérisé en ce que l'on utilise une protéine rendue hydrophobe.

**6.** Procédé selon la revendication 5, caractérisé en ce que la protéine est rendue hydrophobe avant le couplage au moyen de la chaleur, d'un traitement avec des acides, d'agents dénaturants ou d'ions chaotropes et/ou par couplage chimique avec un composé hydrophobe.

**7.** Procédé selon la revendication 4, caractérisé en ce que l'on utilise comme composé difonctionnel le disuccinimidylsubérate.

**8.** Procédé selon l'une des revendications 3 à 7, caractérisé en ce que l'on utilise comme protéine la sérumalbumine bovine, la lipase et/ou l'immuno-$\gamma$-globuline.

**9.** Matériau support utilisable pour les dosages immunologiques en phase solide, qui peut être obtenu selon l'une des revendications 1 à 8, caractérisé en ce qu'il consiste en une phase solide hydrophobe sur laquelle est adsorbé un conjugué constitué par une protéine hydrophobe d'un poids moléculaire supérieur à environ 500000 et une substance protéique capable de liaison spécifique.

**10.** Matériau support selon la revendication 9, caractérisé en ce que la phase solide hydrophobe consiste en polystyrène, en polyméthacrylate, en polyamide, en Teflon® ou en un copolymère de styrène et d'acrylonitrile.

**11.** Matériau support selon la revendication 9 ou 10, caractérisé en ce que la protéine est la sérumalbumine bovine rendue hydrophobe (thermo-RSA), la lipase rendue hydrophobe ou l'immuno-$\gamma$-globuline rendue hydrophobe.

**12.** Matériau support selon l'une des revendications 9 à 11, caractérisé en ce que la substance capable de liaison spécifique est un antigène ou un anticorps.

**13.** Matériau support selon l'une des revendications 9 à 11, caractérisé en ce que la substance capable de liaison spécifique est la streptavidine ou l'avidine.

**14.** Matériau support selon l'une des revendications 9 à 13, caractérisé en ce que le support est un feutre de fibres de verre ou un feutre de libres de cellulose/ester cellulosique et de fibres de polymère.

# FIG.1

FIG . 2

# FIG . 3

T3 – Test

Extinktion am Eppendorf be 405 NM
T3–Standardkonzentration
ng/ml

# FIG.4

HBsAg – Test

Extinktion am Eppendorf bei 405 NM

HBsAg – ad/ay – Standardkonzentration

□ ad    ⊙ ay